(19) Europäisches Patentamt
European Patent Office
Office européen des brevets

(11) EP 2 357 171 A1

(12) EUROPEAN PATENT APPLICATION
published in accordance with Art. 153(4) EPC

(43) Date of publication:
**17.08.2011 Bulletin 2011/33**

(21) Application number: **09823546.8**

(22) Date of filing: **26.10.2009**

(51) Int Cl.:
***C07D 211/14*** (2006.01) ***A61K 31/451*** (2006.01)
***A61K 31/495*** (2006.01) ***A61P 9/10*** (2006.01)
***A61P 25/00*** (2006.01) ***A61P 25/14*** (2006.01)
***A61P 25/16*** (2006.01) ***A61P 25/28*** (2006.01)
***A61P 43/00*** (2006.01) ***C07D 295/02*** (2006.01)

(86) International application number:
**PCT/JP2009/068341**

(87) International publication number:
**WO 2010/050436 (06.05.2010 Gazette 2010/18)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **27.10.2008 JP 2008276046**

(71) Applicant: **M's Science Corporation**
**Hyogo 650-0047 (JP)**

(72) Inventors:
- **MITA, Shiro**
  **Nishinomiya-shi**
  **Hyogo 662-0061 (JP)**
- **KOBAYASHI, Naoyuki**
  **Nara-shi**
  **Nara 631-0032 (JP)**

(74) Representative: **Vossius & Partner**
**Siebertstrasse 4**
**81675 München (DE)**

# (54) PIPERIDINE DERIVATIVE, PIPERAZINE DERIVATIVE AND AGENT FOR TREATMENT OF DISEASE INVOLVING CENTRAL NERVOUS SYSTEM DISORDERS

(57) A novel piperidine derivative and a novel piperazine derivative which are useful as novel sigma receptor ligands, and an agent for treatment of diseases involving a central nervous system disorders, said agent containing one of the compound as an active ingredient. The agent contains a compound represented by general formula (I) or a pharmacologically acceptable salt thereof as an active ingredient:

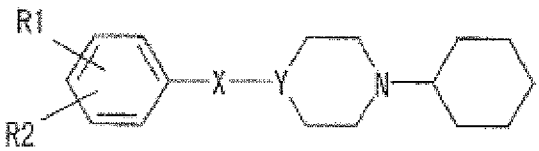

(I)

wherein X represents an alkylene group having to 5 carbon atoms, and the alkylene group may be substituted by one or more alkyl groups each having 1 to 2 carbon atoms; Y represents a carbon atom or a nitrogen atom; and R1 and R2 each independently represents a hydrogen atom, a halogen atom, an alkyl group having 1 to 2 carbon atoms or an alkoxy group having 1 to 2 carbon atoms.

EP 2 357 171 A1

## Description

### TECHNICAL FIELD

The present invention relates to a novel piperidine derivative and a novel piperazine derivative which are useful as novel sigma receptor ligands, and a therapeutic agent of a disease accompanied by a central nervous system disorder. More particularly, it relates to a novel piperidine derivative and a novel piperazine derivative; a pharmaceutical composition for treatment or prevention of symptoms of the diseases, containing the compound as an active ingredient; and a method for treatment or prevention of a disease accompanied by a central nervous system disorder comprising administering the active ingredient.

### BACKGROUND ART

Sigma receptor ligands are known to play a variety of physiological functions by acting on sigma receptors present in central neural cells, etc. Two subtypes of the sigma receptors are currently known, and, among them, a sigma-1 receptor is known to be mainly accompanied by the central nervous system from studies such as pharmacological studies, ethological studies. Recently, it is known that the sigma-1 receptor is involved in various central nervous system disorders including schizophrenia, dementia, depression, etc. It is expected that a compound having a strong affinity to sigma receptors is useful as a therapeutic agent for treatment of a disease accompanied by a central nervous system disorder (for example, schizophrenia, dementia, depression, Parkinson's disease, Huntington's disease, and multiple sclerosis).

According to the recent studies, a finding has been obtained that pharmacological effects related to sigma receptors are based on the promotion of regeneration/maturation of the nerve tissue in association with the sigma receptors. In other words, for example, Patent Document suggests that, in cerebral infarction model rats artificially created by ligating the middle cerebral artery to stop the blood flow, sigma receptors are involved in motor function recovery from cranial neuropathy, as well as suggests that compounds shawing a strong affinity to sigma receptors may be useful as a therapeutic agent for the central nervous system disorder accompanied by cerebral infarction, cerebral contusion, spinal cord injury, etc.

It is known that certain piperidine derivative and piperazine derivative act as a sigma receptor ligand. As these compounds, for example, piperazine derivatives having a phenylalkyl-derived group are disclosed in Patent Document 1, and piperazine derivatives and piperidine derivatives each having a phenylalkyl-derived group are disclosed in Patent Document 2, respectively. However, piperazine derivatives and piperidine derivatives each having an N-substituted cyclohexyl ring and a phenylalkyl-derived group have not been disclosed yet. Moreover, there are not many known compounds that have a high affinity to sigma receptors and promote the regeneration/maturation of the central neural cells as well.

### PRIOR ART DOCUMENTS

#### Patent Documents

Patent Document 1: International Publication WO 2007/021545 Pamphlet
Patent Document 2: International Publication WO 2007/089462 Pamphlet

### SUMMARY OF THE INVENTION

#### PROBLEMS TO BE SOLVED BY THE INVENTION

In view of the current situation described above, the problem of the present invention is to provide a novel piperidine derivative and a novel piperazine derivative as a novel sigma receptor, and a therapeutic agent of a disease accompanied by a central nervous system disorder containing the compound as an active ingredient. Among others, the problem of the present invention is to provide a novel compound that has a high affinity to sigma receptors and promote the regeneration and maturation of central neural cells, as well as to provide a method for treatment or prevention of the disease.

MEANS TO SOLVE THE PROBLEMS

As a result of intensive investigations on compounds in order to solve the above problems, the present inventor has discovered a novel phenylalkyl piperidine derivative or a novel phenylalkyl piperazine derivative each having an N-substituted-cyclohexylalkyl group, which is considered to be a sigma receptor agonist. That is, the present invention relates to a compound represented by the following general formula (I) or a pharmacologically acceptable salt thereof.
(Chemical formula 1)

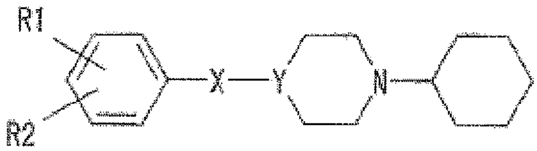

(I)

In the formula, X represents an alkylene group having 1 to 5 carbon atoms, and the alkylene group is optionally substituted by one or more alkyl groups each having 1 to 2 carbon atoms; Y represents a carbon atom or a nitrogen atom; and R1 and R2 each independently represents a hydrogen atom, a halogen atom, an alkyl group having 1 to 2 carbon atoms or an alkoxy group having 1 to 2 carbon atoms.
In one aspect of the present invention, the above compound is one having the formula (I) wherein X represents an alkylene group having 1 to 5 carbon atoms, and the alkylene group is optionally substituted by one or more alkyl groups each having 1 to 2 carbon atoms; Y represents a carbon atom or a nitrogen atom; and R1 and R2 each independently represents a hydrogen atom, or an alkyl group having 1 to 2 carbon atoms.
In another aspect of the present invention, the compound is one which is any one of the following compounds (1) to (6) (referred to as compound (1), compound (6) and the like, respectively), or a pharmacologically acceptable salt thereof:

(1) 1-benzyl-4-cyclohexylpiperazine,
(2) 1-(2-phenylethyl)-4-cyclohexylpiperazine,
(3) 1-(3-phenylpropyl)-4-cyclohexyl-piperazine,
(4) 1-cyclohexyl-4-benzylpiperidine,
(5) 1-cyclohexyl-4-(2-phenylethyl)piperidine, and
(6) 1-cyclohexyl-4-(3-phenylpropyl)piperidine.

The present invention also relates to a pharmaceutical composition for treatment or prevention of a disease accompanied by a central nervous system disorder, containing as an active ingredient at least one of the compound represented by the following general formula (I') and a pharmacologically acceptable salt thereof.
(Chemical formula 2)

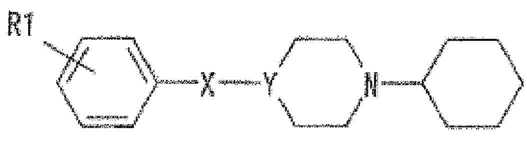

(I')

In the formula, X represents an alkylene group having 1 to 5 carbon atoms, and the alkylene group is optionally substituted by one or more alkyl groups each having 1 to 2 carbon atoms; Y represents a carbon atom or a nitrogen atom; and R1 represents a hydrogen atom or an alkyl group having 1 to 2 carbon atoms.

In one aspect of the present invention, the composition containes at least one of the compounds (1), (2), (3), (4), (5) and (6), and pharmacologically acceptable salts thereof, especially at least one of the compounds (5) and (6), and pharmacologically acceptable salts thereof, as an active ingredient.

Another aspect of the present invention is a method for treatment or prevention of a disease accompanied by a central nervous system disorder, characterized by administering a therapeutically or preventively effective amount of at least one of the compound represented by the general formula (I) and a pharmacologically acceptable salt thereof, for the disease accompanied by central nervous system disorders, to a subject in need of the treatment or prevention. Another further aspect of the present invention is use of at least one of the compound represented by the general formula (I) and a pharmacologically acceptable salt thereof as an active ingredient for the production of a pharmaceutical composition for treatment or prevention of a disease accompanied by a central nervous system disorder.

EFFECT OF THE INVENTION

The compound of the present invention and the pharmacologically acceptable salt thereof (hereinafter also referred to merely as a compound of the present invention) each has a high affinity to sigma receptors, especially sigma-1 receptors. Also, the compound of the present invention has a neurite outgrowth-promoting activity in central neural cells. Based on these pharmacological actions, the compound of the present invention is applicable as a pharmaceutical composition for treatment or prevention of a disease accompanied by a central nervous system disorder, particularly as a pharmaceutical composition effective for nerve regeneration or neuronal protection, for example, as a medicine for treatment or prevention of a disease accompanied by cranial neuropathy (e.g., Alzheimer's disease, Parkinson's disease, Huntington's s disease, sequelae of cranial neuropathy due to cerebrovascular disorder, dementia, disease accompanied by cranial neuropathy due to head injury, disease accompanied by motor dysfunction due to spinal cord injury, and spinal cord damage).

MODE FOR CARRYING OUT THE INVENTION

The compound represented by the general formula (I) is not particularly limited, but includes, for example, a compound of the general formula (I) wherein R1 and R2 each independently represents a hydrogen atom, or an alkyl group having 1 to 2 carbon atoms, and a compound represented by the general formula (I') . In the compound represented by the general formula (I), or where appropriate, by the general formula (I'), X in the formulae represents an alkylene group having 1 to 5 carbon atoms, and the alkylene group is optionally substituted by one or more alkyl groups each having to 2 carbon atoms. The alkylene group having 1 to 5 carbon atoms (e.g., $-CH_2-$, $-CH_2-CH_2-$, $-CH_2-CH_2$ $-CH_2--CH_2-(CH_2-CH_2-CH_2-$, and $-CH_2-CH_2-CH_2-CH_2-CH_2-$) is optionally substituted by one or more alkyl groups each having 1 to 2 carbon atoms. X is preferably an unsubstituted alkylene group, and more preferably an unsubstituted alkylene group having 1 to 3 carbon atoms.

R1 and R2 each independently represents a hydrogen atom, a halogen atom (optionally fluorine, chlorine, bromine, iodine or astatine, typically fluorine, chlorine, bromine, or iodine, preferably fluorine or chlorine, and more preferably fluorine), an alkyl group having 1 to 2 carbon atoms (methyl, ethyl) or an alkoxy group having 1 to 2 carbon atoms (methoxy, methoxy). The substitution positions of R1 and R2 are 2- to 6-positions, especially 3- to 5-positions, further especially 3- or 4-position of the aromatic ring. R1 and R2 each independently is preferably a hydrogen atom or an alkyl group having 1 to 2 carbon atoms and is more preferably a hydrogen atom.

Such a compound is concretely illustrated, and examples preferably include the compounds (1) to (6). The compounds (5) and (6) are more preferable.

The pharmacologically acceptable salt is not particularly limited, but includes, for example, salts with inorganic acids (e.g., sulfuric acid, hydrochloric acid, and phosphoric acid) or organic acids (e.g., acetic acid, oxalic acid, lactic acid, tartaric acid, fumaric acid, maleic acid, trifluoroacetic acid, and methanesulfonic acid), and hydrochloride and tartrate are preferred.

As a method for synthesizing the compound of the present invention, for example, the method in the following scheme 1 can be exemplified for the compound wherein Y is a carbon atom.

(Chemical formula 3)

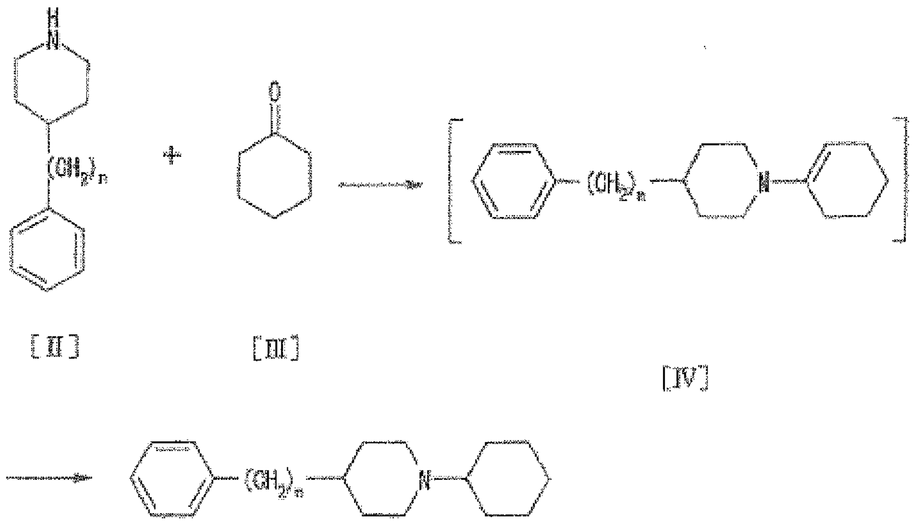

[II] [III] [IV]

[A]

Scheme 1

This method includes a reductive amination synthesis with a 4-phenylalkylpiperidine, which is the compound of the formula [II], or its derivative, namely a compound having a substituent (s) (substituents represented by R1 and R2) on the aromatic ring, and with cyclohexanone which is the compound of the formula [III]. That is, a 4-phenylalkylpiperidine or its derivative is reacted with cyclohexanone to form an enamine ([IV]), which is then reduced with sodium cyanoborohydride, etc. preferably without isolation, thereby to obtain a compound [A] of the present invention (wherein Y is a carbon atom).

The reaction in the above step can be performed in an inert solvent, for example, methanol, at room temperature to 60°C, preferably room temperature, for 5 to 36 hours, preferably 12 to 24 hours, with stirring.

As a method for synthesizing the compound of the present invention, for example, the method in the following scheme 2 can be exemplified for the compound wherein Y is a nitrogen atom.

(Chemical formula 4)

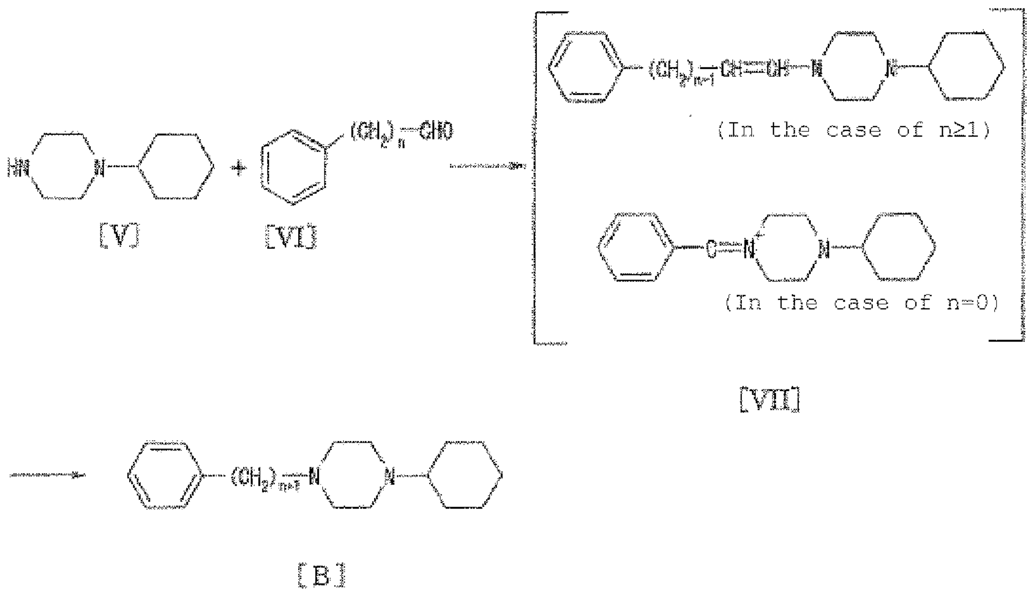


Scheme 2

This method includes a reductive amination synthesis with N-cyclohexylpiperazine which is the compound of the formula [V], and an aldehyde of the formula [VI] or its derivative, namely a compound having a substituent (s) (substituents represented by R1 and R2) on the aromatic ring. That is, N-cyclohexylpiperazine is reacted with an aldehyde or its derivative to form an enamine or iminium ([VII]), and the enamine or iminium is reduced with sodium triacetoxyborohydride, etc., preferably without isolation, thereby to obtain a compound [B] of the present invention (wherein Y is a nitrogen atom).

The reaction in the above step can be performed in an inert solvent, for example, methanol, at room temperature to 60°C, preferably room temperature, for 5 to 36 hours, preferably 12 to 24 hours, with stirring.

The compounds obtained by the methods of the scheme 1 and scheme 2 can be converted into various salts by the conventional method.

In addition, there may be optical isomers in the compound of the general formula [1], but they are all included in the present invention.

Formulation method of pharmaceutical composition of the present invention

The pharmaceutical composition of the present invention contains as an active ingredient at least one of the compound represented by the general formula (I') and a pharmacologically acceptable salt thereof, for example, at least one of the compounds (1) to (6) and pharmacologically acceptable salts thereof, especially at least one of the compounds (5) and (6) and pharmacologically acceptable salts thereof. The dosage form to be used four administration includes, for example, tablets, granules, powders, capsules, syrups, suspensions, and injections. These dosage forms can be prepared using a technique frequently used as a usual formulation method, and, the active ingredients can be formulated into, for example, oral preparations such as tablets, capsules, and granules, using, where necessary, fillers (e.g., lactose, starch, crystalline cellulose, and vegetable oil), lubricants (e.g., magnesium stearate and talc), binders (e.g., hydroxypropyl cellulose and polyvinylpyrrolidone), disintegrators (e.g., carboxymethyl cellulose calcium), coating agents (e.g., hydroxypropyl cellulose, macrogol, and silicone resins), gelatin film-forming agents, syrups, suspensions, and injectable purified water solutions.

For example, in the case of capsules, the active ingredient is contained in the dosage unit amount of, for example, 10 mg, 20 mg, 50 mg, or 100 mg in the form of, for example, a powder, and the above filler, if necessary, may be incorporated in an amount of, for example, 10 mg, 20 mg, 50 mg, or 100 mg. In the case of tablets, the active ingredient in the dosage unit amount of, for example, 0.1 mg, 1 mg, 2 mg, 5 mg, or 10 mg may be incorporated with the filler in an amount of, for example, 20 to 150 mg. In the case of injections, the active ingredient in the dosage unit amount of, for example, 10 mg, 50 mg, or 100 mg may be incorporated with distilled water.

Administration method of pharmaceutical composition of the present invention

The administration of the pharmaceutical composition of the present invention may be performed orally (for example, administration in the dosage form of tablets, granules, powders, capsules, syrups, or suspensions) or parenterally (for example, administration with injections). These administration methods and dosage forms can be appropriately selected depending on the symptom, age, therapeutic purpose, etc. of a subject to be administered in need of treatment or prevention.

The therapeutically or preventively effective amount of the active ingredient in the pharmaceutical composition of the present invention is appropriately selected depending on the symptom, age, dosage form, administration route, therapeutic purpose, etc., but, in the case of human adults, it is 0.01 to 100 mg/individual per day and can be increased or decreased depending on the symptom, age, body weight, race, therapeutic purpose, etc. of a patient. The therapeutically or preventively effective amount of the active ingredient is administered once to several times per day. A single unit dose for human adults is administered, for example, in preferably 0.01 to 40 mg/individual, more preferably 0.1 to 10 mg/individual. The therapeutically or preventively effective amount of the active ingredient is an amount effective for nerve regeneration or neuronal protection. Here, it should be understood that the neuronal protection means the treatment or prevention of nerve injury by regeneration/maturation of nerve cells and preferably promotion thereof . Thus, optimal concentration should be investigated, for example, in the case of compounds where an effect of promoting regeneration/ maturation of neural cells is rather decreased at a thigh concentration. The amount effective for nerve regeneration or neuronal protection can be known by examining the concentration dependency of the pharmacological effects such as neurite outgrowth-promoting activity. This amount varies depending on the compounds, and the effective amount of the active ingredient can be easily set by those skilled in the art because the concentration dependency of the pharmacological action is proved by an evaluation method mentioned below. Further, it is to be mentioned that even compounds with a high affinity to sigma receptors do not always have a neurite outgrowth-promoting activity.

The pharmaceutical composition of the present invention can be applied for treatment or prevention of a disease accompanied by a central nervous system disorder. The disease accompanied by a central nervous system disorder is not particularly limited, but includes typically Alzheimer's disease, Parkinson's disease, Huntington's disease, sequelae of cranial neuropathy due to cerebrovascular disorder, dementia, disease accompanied by cranial neuropathy due to head injury, and disease accompanied by motor dysfunction due to spinal cord injury. A subject that needs the treatment or prevention is usually a human subject, but application to non-human animals that need the treatment or prevention (mammals, for example, non-human primates such as monkey, gorilla, and chimpanzee, small animals (e.g., dog, cat, and mouse), livestock (e.g., cow, horse, and sheep)) is also possible.

Examples

Hereinafter, the present invention will be described in more detail by way of Examples, but it is not intended to be limited thereto.

Example 1

Synthesis of 1-benzyl-4-cyclohexylpiperazine hydrochloride

(Chemical formula 5)

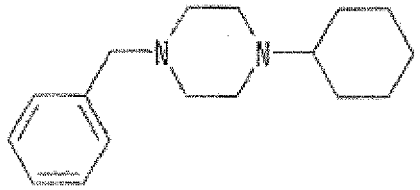

(1)

A methanol (100 mL) solution of N-cyclohexylpiperazine (7. 9 g) and benzaldehyde (5.0g) was stirred at room

temperature for one hour under an argon atmosphere. Subsequently, the reaction solution was cooled with ice, and sodium triacetoxyborohydride (21.2 g) was added at 15°C or less. After returning to room temperature, the solution was stirred overnight. After addition of dichloromethane (300 mL) to the reaction mixture, it was washed with a 10% brine solution, dried over anhydrous magnesium sulfate, and then concentrated under reduced pressure. The resulting oil was purified by a silica gel column chromatography to obtain 1-benzyl-4-cyclohexylpiperazine as an oil. The obtained 1-benzyl-4-cyclohexylpiperazine was further dissolved in a mixture of ethyl acetate (100 mL) and Dichloromethane (100 mL), and 4N hydrogen chloride/ethyl acetate solution (10 mL) was dropwise added thereto under ice-cooling. After returning to room temperature, the solution was stirred for one hour. The precipitated crystal was collected by filtration, washed successively with ethyl acetate and n-hexane, and dried to obtain 1-benzyl-4-cyclohexylpiperazine hydrochloride (8.3 g) as a white crystalline powder.

1H-NMR(DMSO-$d_6$) δ(ppm) ; 1.03 -1.10 (1H,m), 1.21-1.28 (2H,m), 1.38-1.44 (2H,m), 1.58-1.60 (1H,m), 79-1.81 (2H,m), 2.05-2.07 (2H,m), 3.17-3.21(1H,m),3.39-3.62(8H,m),4.01-(1H,bs),4.31-4.51 (2H,m) 7.44-7.48(3H,m),7.64-7.66 (2H,m)

IR (ATR, $cm^{-1}$i) ; 2986, 2936, 2861, 2639, 2511, 2323, 1453, 1433, 1371, 1 080, 1031, 752, 705, 678

Example 2

Synthesis of 1-(2-phenylethyl)-4-cyclohexylpiperazine hydrochloride

(Chemical formula 6)

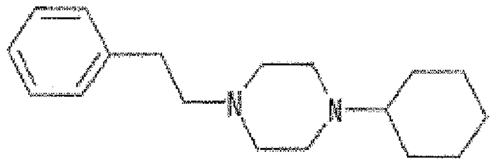

(2)

Methanol (100 mL) solution of N-cyclohexylpiperazine (7.0 g) and phenylacetaldehyde (5.0 g) was stirred at room temperature for one hour under an argon atmosphere. Subsequently, the reaction solution was cooled with ice, and sodium triacetoxyborohydride (18.8 g) was added at 15°C or less. After returning to room temperature, the solution was stirred overnight. Dichloromethane (300 mL) was added to the reaction mixture, and it was washed with a 10% brine solution, dried over anhydrous magnesium sulfate, and then concentrated under reduced pressure. The resulting oil was purified by a silica gel column chromatography to obtain 1-(2-phenylethyl)-4-cyclohexylpiperazine as an oil. The obtained 1-(2-phenylethyl)-4-cyclohexylpiperazine was dissolved in a mixture of ethyl acetate (100 mL) and dichloromethane (100 mL), and 4N hydrogen chloride/ethyl acetate solution (8.9 mL) was dropwise added thereto under ice-cooling. After returning to room temperature, the solution was stirred for one hour. The precipitated crystal was collected by filtration, washed successively with ethyl acetate and n-hexane, and dried to obtain 1-(2-phenylethyl)-4-cyclohexylpiperazine hydrochloride (8.3 g) as a white crystalline powder.

1H-NMR(DMSO-$d_6$) δ(ppm) ;1.02-1.64(6H,m) 1.78-1.84(2H,m)2.08-2.14(2H,m),3.05-3.85(14H,m),-7.25-7.38 (5H,m)

IR(ATR,$cm^{-1}$);2923, 2623, 2497, 2355, 1736, 1455, 1073, 749

Example 3

Synthesis of 1-(3-phenylpropyl)-4-cyclohexyliperazine hydrochloride

(Chemical formula 7)

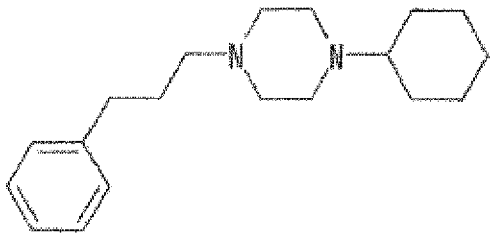

(3)

A methanol (100 mL) solution of N-cyclohexylpiperazine (6.3 g) and 3-phenylpropionaldehyde (5.0 g) was stirred at room temperature for one hour under an argon atmosphere. Subsequently, the reaction solution was cooled with ice, and sodium triacetoxyborohydride (16.9 g) was added at 15°C or less. After returning to room temperature, the solution was stirred overnight. Dichloromethane (300 mL) was added to the reaction mixture, and it was washed with a 10 brine solution, dried over anhydrous magnesium sulfate, and then concentrated under reduced pressure. The resulting oil was purified by a silica gel column chromatography to obtain 1-(3-phenylpropyl)-4-cyclohexylpiperazine as an oil. The obtained 1-(3-phenylpropyl)-4-cyclohexylpiperazine was further dissolved in a mixture of ethyl acetate (100 mL) and dichloromethane (100 mL), and 4N hydrogen chloride/ethyl acetate solution (8.0 mL) was dropwise added thereto under ice-cooling. After returning to room temperature, the mixture was stirred for one hour. The precipitated crystal was collected by filtration, washed successively with ethyl acetate and n-hexane, and dried to obtain 1-(3-phenylpropyl)-4-cyclohexylpiperazine hydrochloride (8.3 g) as a white crystalline powder.

1H-NMR (DMSO-$d_6$)δ(ppm); 0.99-1.49 (5H,m),1.51-1.62(1H,m), 1.75-1.88(2H,m),1.92-2.14(4H,m),2.60-2.66 (2H,m),3.01-3.84(13H,m) 7.15-7.33(5H,m)

IR (ATR,$cm^{-1}$); 2937, 2613, 2502, 2438, 1734, 1447, 1374, 1275, 1076, 1 024,763,721,640

Example 4

Synthesis of 1-cyclohexyl-4-benzylpiperidine hydrochloride

(Chemical formula 8)

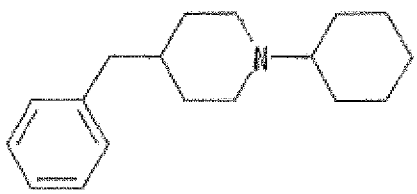

(4)

Methanol (100 mL) solution of 4-benzylpiperidine (5.0 g) and cyclohexanone (2.8 g) was stirred at room temperature for one hour under an argon atmosphere. Subsequently, the reaction solution was cooled with ice, and sodium cyanoborohydride (5.4 g) was added at 15°C or less. The mixture was warmed to 60°C, and stirred for 4 hours. After returning to room temperature, the mixture was further stirred overnight. Dichloromethane (300 mL) was added to the reaction mixture, and it was washed with a 10% brine solution, dried over anhydrous magnesium sulfate, and then concentrated under reduced pressure. The resulting oil was purified by a silica gel column chromatography to obtain 1-cyclohexyl-4-benzylpiperidine as an oil. The obtained 1-cyclohexyl-4-benzylpiperidine was further dissolved in a mixture of ethyl acetate (50 mL) and dichloromethane (50 mL), and 4N hydrogen chloride/ethyl acetate solution (4.0 mL) was dropwise added thereto under ice-cooling. After returning to room temperature, the mixture was stirred for one hour. The precipitated crystal was collected by filtration, washed successively with ethyl acetate and n-hexane, and dried to obtain 1-cyclohexyl-4-benzylpiperidine hydrochloride (3.0 g) as a white crystalline powder.

1H-NMR (DMSO-$d_6$) δ (ppm) ; 0.96-1.41 (5H,m),1.54-1.92 (8H,m), 1.96-2.17(2H,m),2.53(2H,m),2.7-2.94(2H,m),2.95-3.14(1H,m),3.25-3 .88 (2H,m), 7.16-7.32 (5H,m)

IR(ATR, $cm^{-1}$) ; 2929, 2856, 2684, 1743, 1604, 1495, 1454, 1063, 1020, 9 73, 938, 740, 698

Example 5

Synthesis of 1-cyclohexyl-4-(2-phenylethyl)piperidine hydrochloride

(Chemical formula 9)

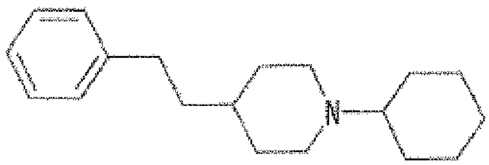

(5)

A methanol (100 mL) solution of 4-(2-phenylethyl)piperidine (5.0 g) and cyclohexanone (2.8 g) was stirred at room temperature for one hour under an argon atmosphere. Subsequently, the reaction solution was cooled with ice, and sodium cyanoborohydride (5.0 g) was added at 15°C or less. The mixture was warmed to 60°C, and stirred for 4 hours. After returning to room temperature, the mixture was further stirred overnight. Dichloromethane (300 mL) was added to the reaction mixture, and it was washed with a 10% brine solution, dried over anhydrous magnesium sulfate, and then concentrated under reduced pressure. The resulting oil was purified by a silica gel column chromatography to obtain 1-cyclohexyl-4-(2-phenylethyl)piperidine as an oil. The obtained 1-cyolohexyl-4-(2-phenylethyl)piperidine was further dissolved in a mixture of ethyl acetate (50 mL) and dichloromethane (50 mL), and 4N hydrogen chloride/ethyl acetate solution (2.5 mL) was dropwise added thereto under ice-cooling. After returning to room temperature, the solution was stirred for one hour. The precipitated crystal was collected by filtration, washed successively with ethyl acetate and n-hexane, and dried to obtain 1-cyclchexyl-4- (2-phenylethyl) piperidine hydrochloride (2.7 g) as a white crystalline powder.

1H-NMR(DMSO-$d_6$) δ(ppm) ; 1.02-1.64 (6H,m), 1.78-1.84 (2H,m), 2.08-2.14 (2H,m),3.05-3.85 (14H,m), 7.25-7.38(5H,m)

IR(ATR,$cm^{-1}$) ; 2922, 2622, 2497, 2355,1735, 1455, 1073, 749, 697

Example 6

Synthesis of 1-cyclohexyl-4-(3-phenylpropyl)piperidine hydrochloride

(Chemical formula 10)

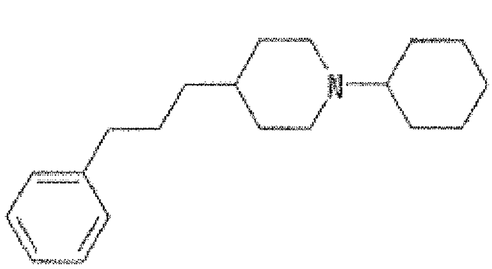

(6)

A methanol (100 mL) solution of 4-(3-phenylpropyl)piperidine (5.0 g) and cyclohexanone (2.5 g) was stirred at room temperature for one hour under an argon atmosphere. Subsequently, the reaction mixture was cooled with ice, and sodium cyanoborohydride (4.8 g) was added at 15°C or less. The mixture was warmed to 60°C, and stirred for 4

hours. After returning to room temperature, the mixture was further stirred overnight. Dichloromethane (300 mL) was added to the reaction mixture, and it was washed with a 10% brine solution, dried over anhydrous magnesium sulfate, and then concentrated under reduced pressure. The resulting oil was purified by a silica gel column chromatography to obtain 1-cyclohexyl-4-(3-phenylpropyl)piperidine as an oil. The obtained 1-oyclohexyl-4-(3-phenylpropyl)piperidine was further dissolved in a mixture of ethyl acetate (50 mL) and dichloromethane (50 mL), and 4N hydrogen chloride/ethyl acetate solution (1.6mL) was dropwise added thereto under ice-cooling. After returning to room temperature, the solution was stirred for one hour. The precipitated crystal was collected by filtration, washed successively with ethyl acetate and n-hexane, and dried to obtain 1-cyclohexyl-4-(3-phenylpropyl) piperidine hydrochloride (1.8 g) as a white crystalline powder.

1H-NMR (DMSO-$d_6$) $\delta$ (ppm) : 0.97-1.65 (11H, m), 1.72-1.86 (4H, m), 2.02 -2.12 (2H,m), 2.51-2.61 (2H,m), 2.78-2.92 (2H,m), 2.99-3.15 (1H,m) ,3.25-3.35(2H,m)7.16-7.30(5H,m)

IR(ATR,$cm^{-1}$) ;2933,2858,2681,2360,1604,1496,1454,1068,1032, 9 67, 940, 745, 715, 694, 668

Pharmacological test of active ingredient in pharmaceutical composition of the present invention

1. Evaluation of affinity to sigma receptors

A membrane preparation as a sigma receptor obtained from the whole brain of guinea pigs was used as an assay system; [$^3$H] (+) -pentazocine was used as a radioactive ligand; (+) -pentazocine was used as a positive substance; and 50 mM Tris hydrochloric acid buffer (pH 7.4) was used as a buffer. In addition, compounds (1) to (6) were used as test substances. A sample was prepared by weighing each of the test substances, a reference compound (SA4503) or a positive substance (haloperidol), and adjusting the final concentrations of the test substances, reference compound and positive substance as shown below with a buffer.
Concentrations (mol/L): $1 \times 10^{-6}$, $1 \times 10^{-7}$, $1 \times 10^{-8}$, $1 \times 10^{-9}$ , $1 \times 10^{-10}$, and $1 \times 10^{-11}$

The inhibition rate and $IC_{50}$ of the test substance, reference compound, and positive substance were determined from a receptor binding experiment by the following method. The measurement of the following radioactivity was performed according to a common method after the binding reaction had been carried out at 25°C for 60 minutes.
The inhibition rate was determined by the equation of "100 - binding rate". The binding rate is represented by the following equation:

$$[(B - N)/(B_0 - N)] \times 100(\%)$$

wherein B is a binding radioactivity to a membrane preparation in the presence of a test substance, a reference compound or a positive substance and a radioactive ligand; $B_0$ is a binding radioactivity (average value) to a membrane preparation in the absence of a test substance, a reference compound, and a positive substance and in the presence of only a radioactive ligand added; and N is a non-specific binding radioactivity (average value).

The $IC_{50}$ of the test substance was calculated from a regression equation to the dose-response curve. That is, the ratio $y=(B-N)/(B_0 - N)$ wherein a value of (B - N) is a specific binding radioactivity in the presence of a test substance, and a value of ($B_0$ - N) is a specific binding radioactivity in the absence of a test substance was first subjected to logit conversion and applied to a logit-log model plotted against the common logarithm of the test substance concentration to create a dose-response curve. In the same way, the $IC_{50}$ values of the reference compound and the positive substance were determined. The regression equation to the dose-response curve used is as follows:

$$Y=aX + b$$

wherein Y = logity = ln(y/(1 - y)), X = log x, and a and b are each a constant, provided that x is a solute concentration of a test substance.

Then, for each concentration of the positive substance, the reaction rate was measured while changing the concentration of the test substance in a range including the $IC_{50}$ to create the Dixon plot, and the Ki value was calculated from the test substance concentration giving an intersection of these straight lines. The results were shown in Table 1.

2. Evaluation of neurite outgrowth-promoting activity in central neural cells

The neurite outgrowth-promoting effect of the rat cerebral cortex neural cells on test substances was evaluated

using the following method. The cerebral cortex neural cells were isolated from Wistar rats on the 18th day of pregnancy according to the method of Springer Neuroscience Lab Manual . That is, under ether anesthesia, a fetal rat was removed from the uterus of a pregnant rat on day 18, and transferred into a petri dish containing HBSS (manufactured by Gibco, Inc.) on ice. The brain was subdivided into three pieces under a stereoscopic microscope to cut out the cerebral cortex. The cerebral cortex that had been cut out was dispersed with a neural cell dispersion solution (manufactured by Sumitomo Bakelite Co. , Ltd.) to prepare rat cerebral cortex cells.

The resulting cerebral cortex cells were subjected to cell culture. Neurobasal medium (manufactured by Gibco, Inc.) supplemented with B27 Supplement (manufactured by Gibco, Inc.; Fin. 2 % v/v), L- glutamine (manufactured by IBCO company; Fin. 0.5 mM) and GlutaMax I (manufactured by Gibco, Inc.; Fin. 0.5 mM) was used as the culture medium. The culture conditions were as follows: 5% $CO_2$, 95% air, 100% humidity, and 37°C. The cells were inoculated to polylysine-coated 8-well slides (manufactured by IWAKI company) so as to have about $2 \times 10^4$ cells/well. Compounds (1) to (6) were added as respective test substances (final concentrations: 0.3 μm and 1.0 μM) to the culture solution at about 3 hours after the cell inoculation, and the culture solution with no additives was used as a control.

At 48 hours after the addition of the compounds, the cells were fixed at room temperature for about 30 minutes using 4% paraformaldehyde (manufactured by Wako Pure Chemical Industries), and incubated with an anti-α-Tublin antibody (Anti-α-Tubulin Alexa Fluor 488 conjugate) recognizing a microtubule protein, at room temperature for one hour. After washing, the nucleus of the whole cell was stained with a nucleic acid-staining agent DAPI. The cells were observed under a fluorescence microscope at a magnification of 20 times. The stained cell image and the nuclear staining image in the same field of vision were imported into a computer as the image. Using an image analysis software (MethaMorph (registered trademark), Molecular Devises), the image imported into the computer, was used to measure the "longest neurite length" that was the length of the longest neur i te of individual cell in each field of vision. A cell having a length of the "longest neurite length" of 60 μm or more is defined as a "neurite forming cell" and the neurite forming activity was evaluated by the ratio of the "neurite forming cell" occupied in the number of the cells in the same field of vision. In other words, such evaluation was performed by determining the rate of the neurite forming cells using the following equation:

$$\text{Rate of neurite forming cells} = [\text{Number of neurite forming cells} / \text{Number of total cells in the same field of vision}] \times 100$$

The results were shown in Table 1. Each numerical value represents mean ± standard error.

[Table 1]

| | Sigma receptor binding activity | Neurite outgrowth-promoting activity (%) | |
|---|---|---|---|
| | Ki(nM) | 0.3 μM | 1.0 μM |
| Reference compound | 7.4 | 44.6 ± 2.2 | 58.4 ± 1.8 |
| Compound (1) | 6.5 | 55.2 ± 1.5 | 54.6 ± 1.5 |
| Compound (2) | 6.0 | 55.7 ± 2.4 | 55.1 ± 2.3 |
| Compound (3) | 6.1 | 49.6 ± 2.7 | 58.4 ± 2.8 |
| Compound (4) | 7.0 | 50.7 ± 2.7 | 51.1 ± 2.9 |
| Compound (5) | 3.9 | 51.2 ± 2.0 | 48.9 ± 1.5 |
| Compound (6) | 2.1 | 54.2 ± 1.7 | 46.0 ± 2.4 |
| Control | - | 18.0 ± 1.6 | |

From the results of Table 1, it was clear that all these compounds of the present invention inhibited a specific binding of a radioactive ligand to a sigma receptor at a very low concentration compared to the reference compound which was a sigma receptor agonist. Accordingly, it was found that all of the above active ingredients in the pharmaceutical compositions of the present invention had a very high affinity to sigma receptors.

In addition, as for the rat cerebral cortex neural cells cultured for 48 hours after addition of each of these

compounds of the present invention, it became clear that the ratio of cells each having a neurite length of 60 μm or more was significantly increased, and thus such compounds were found to clearly have a neurite outgrowth-promoting activity.

Examples of general preparations such as oral preparations and injections of the pharmaceutical compositions according to the present invention are shown below.

Preparation example 1

Tablet

| | |
|---|---|
| Compound (1) of the present invention | 1 mg |
| Lactose | 55 mg |
| Corn starch | 30 mg |
| Carboxymethyl cellulose calcium | 10 mg |
| Hydroxypropyl cellulose | 3 mg |
| Magnesium stearate | 1 mg |
| Total | 100 mg |

A coating was applied to a tablet having the above formulation using a coating agent 2 mg (for example, a usual coating agent such as hydroxymethyl cellulose, macrogol, or a silicone resin), thereby making it possible to obtain the target tablet with coating (the same as in the tablet with the following formulation) . In addition, the target tablet can be obtained by using other compound of the present invention or changing the amount of additives appropriately.

Preparation example 2

Capsule

| | |
|---|---|
| Compound (1) of the present invention | 5 mg |
| Lactose | 145 mg |
| Total | 150 mg |

In addition, the target capsule can be obtained by using other compound of the present invention or changing the amount of an additive appropriately.

Preparation example 3

Injection

| | |
|---|---|
| Compound (1) of the present invention | 10 mg |
| Sodium chloride | 0.9 mg |
| Sodium hydroxide (or hydrochloric acid) | q.s. |
| Sterile purified water | q.s. |
| Total | 10 ml |

In addition, the target injection can be obtained by using otter compound of the present invention or changing the amount of additives appropriately.

## Claims

**1.** A compound represented by the following general formula (I) or a pharmacologically acceptable salt thereof:

(Chemical formula 1)

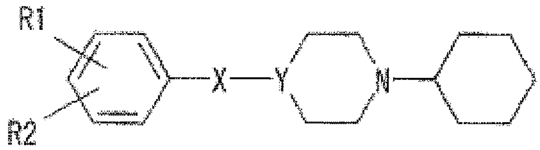

(I)

wherein X represents an alkylene group having to 5 carbon atoms, and the alkylene group is optionally substituted by one or more alkyl groups each having 1 to 2 carbon atoms; Y represents a carbon atom or a nitrogen atom; and R1 and. R2 each independently represents a hydrogen atom, a halogen atom, an alkyl group having to 2 carbon atoms or an alkoxy group having 1 to 2 carbon atoms.

**2.** The compound according to claim 1, wherein R1 and R2 in the general formula (I) each independently represents a hydrogen atom, or an alkyl group having 1 to 2 carbon atoms.

**3.** The compound according to claim 1 or 2, wherein X in the general formula (I) represents an unsubstituted alkylene group having 1 to 5 carbon atoms; Y represents a carbon atom or a nitrogen atom; and R1 and R2 each represents a hydrogen atom.

**4.** The compound according to claim 3, which is any one of the following compounds (1) to (6), or a pharmacologically acceptable salt thereof:

(1) 1-benzyl-4-cyclohexylpiperazine,
(2) 1-(2-phenylethyl)-4-Cyclohexylpiperazine,
(3) 1-(3-phenylpropyl)-4-cyclohexylpiperazine,
(4) 1-cyclohexyl-4-benzylpiperidine,
(5) 1-cyclohexyl-4-(2-phenylethyl)piperidine, and
(6) 1-cyolohexyl-4-(3-phenylpropyl)piperidine.

**5.** A pharmaceutical composition, for treatment or prevention of a disease accompanied by a central nervous system disorder, comprising as an active ingredient at least one of a compound represented by the following general formula (I') and a pharmacologically acceptable salt thereof:

(Chemical formula 2)

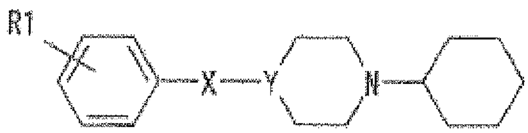

(I')

wherein X represents an alkylene group having 1 to 5 carbon atoms, and the alkylene group is optionally substituted by one or more alkyl groups each having to 2 carbon atoms; Y represents a carbon atom or a nitrogen atom; and R1 represents a hydrogen atom or an alkyl group having 1 to 2 carbon atoms.

**6.** The pharmaceutical composition according to claim 5, wherein X in the general formula (I') represents an unsubstituted alkylene group having 1 to 5 carbon atoms; Y represents a carbon atom or a nitrogen atom; and R1 represents a hydrogen atoms.

**7.** The pharmaceutical composition according to claim 6, which comprises as an active ingredient at least one of the following compounds (1), (2), (3), (4), (5) and (6), and pharmacologically acceptable salts thereof:

(1) 1-benzyl-4-cyclohexylpiperazine,

(2) 1-(2-phenylethyl)-4-cyclohexylpiperazane,
(3) 1-1,3-phenylpropyl)-4-cyclohexylpiperazine,
(4) 1-cyclohexyl-4-benzylpiperidine,
(5) 1-cyclohexyl-4-(2-phenylethyl)piperidine, and
(6) 1-cyclohexyl-4-(3-phenylpropyl)piperidine.

**8.** The pharmaceutical composition according to claim 7, which comprises as an active ingredient at least one of the following compounds (5) and (6), and pharmacologically acceptable salts thereof:

(5) 1-cyclohexyl-4-(2-phenylethyl)piperidine, and
(6) 1-cyclohexyl-4-(3-phenylpropyl)piperidine.

**9.** The pharmaceutical composition according to any one of claims 5 to 8, wherein the disease accompanied by a central nervous system disorder is at least one selected from the group consisting of Alzheimer's disease, Parkinson's disease, Huntington's disease, sequelae of cranial neuropathy due to cerebrovascular disorder, dementia, disease accompanied by cranial neuropathy due to head injury, and disease accompanied by motor dysfunction due to spinal cord injury.

**10.** A method for treatment or prevention of a disease accompanied by a central nervous system disorder, **characterized by** administering a therapeutically or preventively effective amount of at least one of the compound according to any one of claims 1 to 4 and a pharmacologically acceptable salt thereof, for the disease accompanied by a central nervous system disorder, to a subject in need of treatment or prevention of the disease,

**11.** The method according to claim 10, wherein the disease accompanied by a central nervous system disorder is at least one selected from the group consisting of Alzheimer' s disease, Parkinson's disease, Huntington's disease, sequelae of cranial neuropathy due to cerebrovascular disorder, dementia, disease accompanied by cranial neuropathy due to head injury, and disease accompanied by motor dysfunction due to spinal cord injury.

**12.** Use of at least one of any one of the compounds according to claims 1 to 4 and a pharmacologically acceptable salt thereof as an active ingredient in the production of a pharmaceutical composition for treatment or prevention of a disease accompanied by a central nervous system disorder.

**INTERNATIONAL SEARCH REPORT**

International application No.
PCT/JP2009/068341

A. CLASSIFICATION OF SUBJECT MATTER
*C07D211/14*(2006.01)i, *A61K31/451*(2006.01)i, *A61K31/495*(2006.01)i, *A61P9/10*(2006.01)i, *A61P25/00*(2006.01)i, *A61P25/14*(2006.01)i, *A61P25/16*(2006.01)i, *A61P25/28*(2006.01)i, *A61P43/00*(2006.01)i, *C07D295/02*(2006.01)i

According to International Patent Classification (IPC) or to both national classification and IPC

B. FIELDS SEARCHED

Minimum documentation searched (classification system followed by classification symbols)
C07D211/14, A61K31/451, A61K31/495, A61P9/10, A61P25/00, A61P25/14, A61P25/16, A61P25/28, A61P43/00, C07D295/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | NATSUKA, Kagayaki et al., 1-Substituted 4-(1,2-diphenylethyl)-piperazine derivatives and their analgesic activities. 1, Journal of Medicinal Chemistry, 1975, Vol.18, No.12, pp.1240-1244 | 1-4 |
| X | Chemical Abstracts, 1981, Vol.94, p.570, Abstract No.30706 | 1-4 |
| X | Chemical Abstracts, 1975, Vol.83, p.498, Abstract No.58749 | 1-4 |
| X | AT 229873 B (CHINOIN GYOGYSZER ES VEGYESZETI TERMEKEK GYARA R.T.), 25 October 1963 (25.10.1963), entire text; particularly, Beispiel 2 (Family: none) | 1-7,12 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 November, 2009 (24.11.09) | 08 December, 2009 (08.12.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2009/068341

C (Continuation). DOCUMENTS CONSIDERED TO BE RELEVANT

| Category* | Citation of document, with indication, where appropriate, of the relevant passages | Relevant to claim No. |
|---|---|---|
| X | LANYI, Kalman et al., Piperazine derivatives. II. Effect of piperazine derivatives, Arzneimittel-Forschung, 1968, Vol.18, No.11, pp.1431-1435 | 1,2,5,12 |
| X | US 2858313 A (MILES LABORATORIES, INC.), 28 October 1958 (28.10.1958), entire text; particularly, column 1 (Family: none) | 1,2 |
| X | LANYI, Kalman et al., Piperazine derivatives. I. Alkyl-, aryl, aralkyl-piperazine derivatives, Chemische Berichte, 1967, Vol.100, No.9, pp.3045 -3051 | 1,2 |
| X<br>A | WO 2007/021545 A2 (M'S SCIENCE CORP.), 22 February 2007 (22.02.2007), entire text; particularly, claims; page 8, line 27 to page 9, line 12<br>& JP 2009-504648 A & EP 1931347 A2<br>& US 2009/143395 A1 & CA 2617346 A1<br>& AU 2006280203 A1 & CN 101263127 A | 1-7,9,12<br>8 |
| X | JP 11-501014 A (Schering Corp.), 26 January 1999 (26.01.1999), entire text; particularly, pages 13 to 20, 62 to 63<br>& JP 2008-24714 A & WO 96/026196 A2<br>& EP 811002 A1 & ZA 9601293 A<br>& AU 9649717 A & FI 9703446 A<br>& KR 98702509 A & HU 9800792 A<br>& NZ 303415 A & MX 9706381 A<br>& TW 464646 A | 1,2,5,9,12 |

Form PCT/ISA/210 (continuation of second sheet) (April 2007)

**INTERNATIONAL SEARCH REPORT**

International application No.
PCT/JP2009/068341

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 10, 11
because they relate to subject matter not required to be searched by this Authority, namely:
Claims 10 and 11 pertain to methods for treatment of the human body by therapy and thus relate to a subject matter which this International Searching Authority is not required, under the provisions of Rule 39.1(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007021545 A
- WO 2007089462 A